(19) Europäisches Patentamt
European Patent Office
Office européen des brevets

(11) Veröffentlichungsnummer : 0 326 934 B1

(12) # EUROPÄISCHE PATENTSCHRIFT

(45) Veröffentlichungstag der Patentschrift : **30.10.91 Patentblatt 91/44**

(21) Anmeldenummer : **89101310.4**

(22) Anmeldetag : **26.01.89**

(51) Int. Cl.[5] : **C07B 31/00**, // C07C33/22, C07C211/03, C07C215/08, C07C217/16, C07C321/04, C07C381/14, C07D207/08, C07C29/136, C07C209/30

(54) **Verfahren zur Reduktion reduzierbarer Verbindungen.**

(30) Priorität : **02.02.88 DE 3802981**

(43) Veröffentlichungstag der Anmeldung : **09.08.89 Patentblatt 89/32**

(45) Bekanntmachung des Hinweises auf die Patenterteilung : **30.10.91 Patentblatt 91/44**

(84) Benannte Vertragsstaaten : **BE CH DE ES FR GB IT LI NL**

(56) Entgegenhaltungen :
**WO-A-83/00686**
**US-A- 2 856 428**
**US-A- 2 945 886**
**US-A- 3 014 025**

(56) Entgegenhaltungen :
**E. MÜLLER et al.: "Methoden der Organischen Chemie", (Houben-Weyl), Band IV/1d, 4. Auflage, Teil II, 1981, Seite 38, Georg Thieme Verlag, Stuttgart, DE; J. BRACHT et al.: "Reduktion mit Metallhydriden bzw. komplexen Hydriden"**
**Fieser/Fieser, Reagents for Organic Synthesis, John Wiley and Sons, Inc., New York Volume 1, 1967, Seiten 1053-54 Volume 3, 1972, Seiten 264-65 Volume 7, 1979, Seite 322**

(73) Patentinhaber : **BASF Aktiengesellschaft**
**Carl-Bosch-Strasse 38**
**W-6700 Ludwigshafen (DE)**

(72) Erfinder : **Sandhoff, Konrad, Prof. Dr.**
**Auf dem Patt 12**
**W-5305 Alfter-Impekoven (DE)**
Erfinder : **Athanassios, Giannis, Dr.**
**Londoner Strasse 13**
**W-5300 Bonn (DE)**
Erfinder : **Steglich, Wolfgang, Prof. Dr.**
**Hobsweg 77**
**W-5300 Bonn-Roettgen (DE)**

**EP 0 326 934 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Reduktion reduzierbarer Verbindungen mit Hilfe Alkalimetallboranaten in Gegenwart eines komplexierenden Lösungsmittels.

Dieses Reduktionsverfahren, bei dem es sich meistens um die Hydrierung organischer Verbindungen handelt, ist, sieht man von der erfindungsgemäßen Verbesserung ab, allgemein bekannt und läßt sich beispielsweise für den Fall der Überführung eines Ketons in den entsprechenden sekundären Alkohol wie folgt formulieren :

(1)
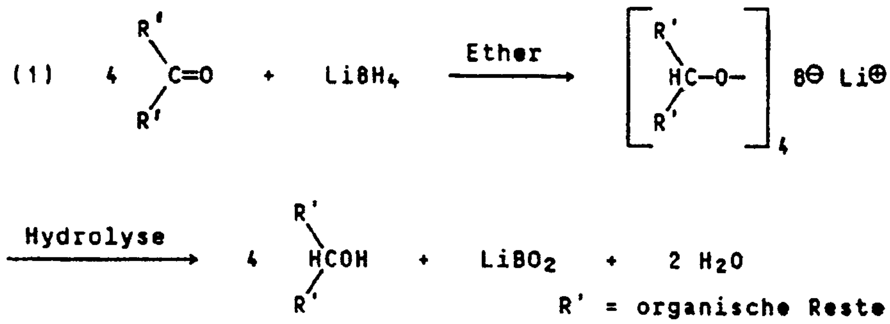


So attraktiv dieses Verfahren auch ist, so hat es doch den Nachteil, daß die Reduktionskraft der Alkalimetallboranate in vielen Fällen nicht ausreicht, weshalb man auf die entsprechenden Alkalimetallalanate ausweichen muß, wie sich der Tabelle 5 auf Seite 1118 der Monographie von Jerry March "Advanced Organic Chemistry : Reactions, Mechanisms and Structure", Second Edition, 1977 (McGraw Hill) entnehmen läßt.

Die Alanate sind zwar wesentlich aktiver als die Boranate, dafür aber teurer und vor allem schwieriger zu handhaben, da sie im Gegensatz zu den Boranaten höchst wasserempfindlich sind und sich bereits durch den Einfluß von Luftfeuchtigkeit unter Freisetzung von Wasserstoff zersetzen, wobei sich mit der Luft hochexplosives Knallgas bildet.

"Aus der Monographie von Fieser-fieser (Reagents for Organic Synthesis, Band 1, 1967, Seiten 1053, 1054) ist es speziell bekannt, als Reduktionsmittel eine Mischung aus Natriumboranat und Aluminiumchlorid zu verwenden. Weiterhin ist es aus den US-A-2856428 und US-A-2945886 bekannt, die Reduktionskraft von Alkkalimetallboranaten mit Borhalogeniden oder Metallchloriden, darunter Aluminiumchlorid, zu verstärken, wobei angenommen wird, daß die Reduktion über Komplexe aus dem Boranat und dem Halogenid verläuft. Schließlich betrifft die WO-83/00686 die Herstellung von Silanen durch Umsetzung von Halogen-silanen mit Natriumboranat, wobei Borane freigesetzt werden."

Der Erfindung lag die Aufgabe zugrunde, die Reduktionswirkung der Alkalimetallboranate durch Mitverwendung eines Reduktionshilfsmittels zu erhöhen und die Alkalimetallboranate somit für solche Reduktionen zugänglich zu machen, für die sie allein nicht oder nur unzureichend geeignet sind.

Demgemäß wurde ein Verfahren zur Reduktion reduzierbarer Verbindungen mit Hilfe von Alkalimetallboranaten in Gegenwart eines komplexierenden Lösungsmittels und eines Reduktionshilfsmittels gefunden, welches dadurch gekennzeichnet ist, daß man als Reduktionshilfsmittel wirksame Mengen eines Alkylhalogensilans der allgemeinen Formel I

$$R_n{-}Si{-}X_{4-n} \quad I$$

mitverwendet, in der R für gleiche oder verschiedene $C_1$-$C_4$-Alkylgruppen steht, X Chlor oder Brom bezeichnet und n einen Wert von 1 bis 3 hat.

Die aktivierende Wirkung der Alkylhalogensilane I beruht vermutlich darauf, daß Boran freigesetzt wird, welches ein stärkeres Reduktionsmittel ist als die Alkalimetallboranate. Es ist daher anzunehmen, daß z.B. im Falle des Trimethylchlorsilans folgende Primärreaktion stattfindet :

$$(2) \quad LiBH_4 + Me_3SiCl \xrightarrow{Ether} [BH_3 \cdot Ether] + Me_3SiH + LiCl$$

Als Alkalimetallboranate kommen aus wirtschaftlichen Gründen in erster Linie das Lithiumboranat und das Natriumboranat in Betracht.

Unter den Alkylhalogensilanen I eignen sich sowohl die Chlor- als auch die Bromverbindungen, jedoch ist den Chlorverbindungen, ebenfalls aus wirtschaftlichen Gründen, generell der Vorzug zu geben.

Besonders glatt verläuft die Reaktion mit Trialkylmonohalogensilanen I (n = 3), jedoch erfüllen auch die Dialkyldihalogensilane (n = 2) und, mit geringen Einschränkungen, die Monoalkyltrihalogensilane (n = 1) den erfindungsgemäßen Zweck.

Auf die Art der Alkylgruppen R in den Verbindungen I kommt es nach den bisherigen Beobachtungen nicht an, so daß man sich hier in erster Linie nach der Verfügbarkeit dieser Verbindungen richten wird. Die größte Bedeutung ist daher derzeit dem Trimethylchlorsilan und dem Dimethyldichlorsilan beizumessen.

Weitere geeignete definitionsgemäße Verbindungen I sind beispielsweise das Triethylchlorsilan und das Tri-n-butylchlorsilan. Verbindungen mit höheren Alkylgruppen, etwa das Tri-n-butylchlorsilan, wird man in verfahrenstechnischer Hinsicht dann in Betracht ziehen, wenn man keine leichtsiedenden Silane wie Trimethylsilan als Nebenprodukte wünscht.

Legt man auf eine vollständige Reduktion der zu reduzierenden Verbindung (Edukt) Wert, so ist das Alkalimetallboranat in mindestens äquivalenten Mengen zur Anzahl der Funktionen des Eduktes einzusetzen, die reduziert werden sollen.

Da das erfindungsgemäß wirksame Agens [$BH_3$ · Ether] gemäß den Gleichungen (1) und (2) drei Reduktions- oder Hydrieräquivalenten entspricht, beträgt das Mindestmolverhältnis von Alkalimetallboranat zu einem monofunktionellen Edukt 0,33 : 1, jedoch ist es häufig zweckmäßig, das Alkalimetallboranat in einem Überschuß, etwa bis zu einem Verhältnis von 4 : 1, anzuwenden, weil diejenige Hydridfunktion des Boranates bzw. des Borans das größte Reduktionspotential hat, die zuerst reagiert, wogegen jede weitere verbleibende Hydridfunktion von schwächerer Wirkung ist.

Die gleichen Empfehlungen gelten für die Menge des Alkylhalogensilans I, welche zur Menge des Alkalimetallboranats äquivalent sein sollte. Ist also n = 3, beträgt das Molverhältnis von I zum Alkalimetallboranat regelmäßig 1 : 1, und es ermäßigt sich auf 0,5 : 1 und 0,33 : 1 für die Fälle n = 2 bzw. n = 1. Allerdings gelingt die Reduktion manchmal auch bereits mit unteräquivalenten Mengen von I (etwa ab 50% der äquivalenten Menge), aber meistens empfehlen sich überäquivalente Mengen bis zu 250%, und zwar vorzugsweise etwa 200%, der äquivalenten Menge.

Die Mitverwendung eines komplexierenden Lösungsmittels hat einen wesentlichen Einfluß auf das Gelingen des Verfahrens. Als komplexierende Lösungsmittel kommen in erster Linie Verbindungen mit Etherstruktur in Betracht, die sich bei der Reduktion inert verhalten, also z.B. Diethylether, 2,2'-Dimethoxydiethylether, Tetrahydropyran und vor allem Tetrahydrofuran.

Auch tertiäre Stickstoffbasen wie Triethylamin und Pyridin sowie Thioether und tertiäre Phosphorverbindungen wie Triphenylphosphit lassen sich vielfach als komplexierende Lösungsmittel verwenden.

Entsprechend ihrer Funktion als komplexierende Verbindungen

(3) 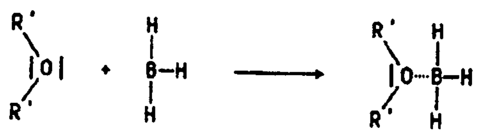

sind diese Lösungsmittel in mindestens äquimolaren Mengen zum Alkalimetallboranat einzusetzen, jedoch ist es meistens zweckmäßig, sie in einem Überschuß zu verwenden, der von der Löslichkeit der zu reduzierenden Verbindung abhängt.

Neben dem komplexierenden Lösungsmittel können noch weitere inerte Lösungsmittel mitverwendet werden, beispielsweise um die Löslichkeit aller Reaktanten zu verbessern, jedoch ist dies im allgemeinen nicht erforderlich.

Grundsätzlich kann man die Reduktionspartner Edukt, Lösungsmittel, Alkalimetallboranat und Alkylhalogensilan I in beliebiger Reihenfolge zueinander geben. In aller Regel ist es aber zweckmäßig, das Alkalimetallboranat zunächst in dem Lösungsmittel zu lösen oder zu suspendieren, danach das Alkylhalogensilan zuzugeben und das Gemisch solange zu rühren, bis der Großteil des Boranats unter Entwicklung des Silans $R_n$–Si–$H_{n-4}$ mit dem Alkylhalogensilan reagiert hat, und das so erhaltene reduktive Agens sodann mit dem Edukt zu versetzen.

Hierbei nimmt man die Bereitung des reduktiven Agens vorzugsweise bei Temperaturen von (–30) bis 140°C, besonders von 0 bis 100°C vor, wonach man die Reduktion des Edukts je nach dessen Reaktivität vorzugsweise bei (–30) bis 140, besonders bei 0 bis 80°C ausführt.

In aller Regel wird man bei Normaldruck oder im Falle tiefsiedender Reaktionspartner unter dem Eigendruck des Systems arbeiten.

Im übrigen kann man das erfindungsgemäße Verfahren auf die gleiche Weise vornehmen wie bei der Verwendung der Alkalimetallboranate allein, so daß nähere Angaben hierzu entbehrlich sind. Das gleiche gilt für die Aufarbeitung der Reaktionsgemische durch Alkoholyse und/oder Hydrolyse gemäß Gleichung (1).

Das erfindungsgemäße Verfahren eignet sich zur Reduktion oder zur reduktiven Spaltung all derjenigen Verbindungen, die man bekanntermaßen mit Lithiumalanat reduzieren bzw. hydrieren kann und bietet besondere Vorteile in den Fällen, in welchen die Reduktionskraft der Alkalimetallboranate für sich allein nicht oder nur ungenügend ausreicht.

Beispiele hierfür sind vor allem die Reduktion

— von Carboxylaten zu Alkoholen

$$R'-COO^{\ominus} \longrightarrow R'-CH_2-OH + OH^{\ominus}$$

— von Estern zu Alkoholen

$$R'-COO-R'' \longrightarrow R'-CH_2OH + R''-OH$$

— von Säurechloriden zu Aldehyden und Alkoholen

$$R'-COCl \longrightarrow R'-CHO \longrightarrow R'-CH_2-OH$$

— von Nitroverbindungen zu Aminen und Azoverbindungen

$$R'-NO_2 \longrightarrow R'-NH_2 \quad \text{oder} \quad R'-N=N-R'$$

— von Säuren zu Alkoholen

$$R'-COOH \longrightarrow R'-CH_2OH$$

— von Säureamiden zu Aminen

$$R'-CO-NR''_2 \longrightarrow R'-CH_2-NR''_2$$

$R''$ = org. Rest; H

— von Nitrilen zu Aminen

$$R'-CN \longrightarrow R'-CH_2-NH_2$$

— von Lactonen zu Diolen

$$\underbrace{R'-CO-O} \longrightarrow HO-R'-CH_2OH$$

— von Lactamen zu Iminen

$$\underbrace{R'-CO-NH} \longrightarrow \underbrace{R'-CH_2-NH}$$

— von Epoxiden zu Alkoholen

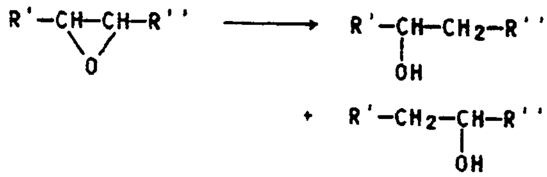

— von Olefinen zu den gesättigten Kohlenwasserstoffen

$$R'\text{—}CH\text{=}CH\text{—}R' \longrightarrow R'\text{—}CH_2\text{—}CH_2\text{—}R'$$

und
— von Sulfonen zu Sulfiden

$$R'\text{—}SO\text{—}R'' \longrightarrow R'\text{—}S\text{—}R''$$

Aber auch in Fällen leichter reduzierbarer Edukte, in denen die Reduktion mit den Alkalimetallboranaten allein gelingt, kann sich das erfindungsgemäße Verfahren empfehlen, sei es, daß die Reaktion hiermit schneller und mit höherer Ausbeute verläuft oder sei es, daß sich bestimmte Verbindungen dieser Klassen der Reduktion z.B. aus sterischen Gründen oder wegen einer inhibierenden Substitution stärker widersetzen.

Derartige Reaktionen sind beispielsweise die Reduktion
— von Aldehyden zu Alkoholen

$$R'\text{—}CHO \longrightarrow R'\text{—}CH_2OH$$

und
— von Ketonen zu sekundären Alkoholen

$$R'\text{—}CO\text{—}R'' \longrightarrow R'\text{—}CHOH\text{—}R''$$

Grundsätzlich kommen weiterhin auch anorganische Reduktionen in Betracht.

Da das erfindungsgemäße Verfahren prinzipiell und universell auf alle Klassen reduzierbarer Verbindungen anwendbar ist, ist eine erschöpfende Aufzählung der in Betracht kommenden Reaktionen und einzelner Verbindungsklassen und Verbindungen weder möglich noch sinnvoll. Für den speziellen Fall hat es der Fachmann unschwer in der Hand, die Eignung des Verfahrens festzustellen und die optimalen Reaktionsbedingungen in einigen Vorversuchen zu ermitteln.

Wegen der schonenden Reaktionsbedingungen empfiehlt sich das Verfahren vor allem für die Reduktion bzw. Hydrierung empfindlicher Substanzen, unter denen optisch aktive Verbindungen, die stets eine gewisse Neigung zu unerwünschter Racemisierung haben, besonders hervorgehoben seien.

Beispiel 1

Reduktion von 3,4-Dimethoxybenzylcyanid

Eine Lösung aus 10 g (56,4 mmol) 3,4-Dimethoxybenzylcyanid, 18,36 g (170 mmol) Trimethylchlorsilan und 100 ml Tetrahydrofuran wurde bei 25°C mit 8,16 g (170 mmol) Natriumboranat versetzt und danach rd. 10 Stunden lang unter Rückflußkühlung zum Sieden (66°C) erhitzt, wobei Trimethylsilan entwich.

Danach wurde das Gemisch der Alkoholyse mit Methanol und, nach destillativer Abtrennung von Methanol

und Tetrahydrofuran, der Hydrolyse mit verdünnter Salzsäure unterworfen.

Die salzsaure Lösung wurde mit Ether gewaschen und sodann mit einem Überschuß verdünnter Natronlauge versetzt, wonach das freigesetzte Amin mit Methylenchlorid extrahiert wurde. Die übliche Aufarbeitung der Extraktphase lieferte das reine 2-(3,4-Dimethoxyphenyl)-ethylamin in der in der Tabelle angegebenen Ausbeute.

Beispiel 2

Reduktion von L-Valin

Eine Lösung aus 0,87 g (40 mmol) Lithiumboranat und 20 ml Tetrahydrofuran wurde im Laufe von 10 min mit 8,64 g (80 mmol) Trimethylchlorsilan versetzt, wobei sich ein Niederschlag von Lithiumchlorid bildete. Zu diesem Gemisch wurden innerhalb von 5 min portionsweise 2,34 g (20 mmol) L-Valin gegeben, worauf es noch 24 h bei 25°C gehalten wurde. Die übliche Aufarbeitung lieferte das L-Valinol in der in der Tabelle angegebenen Ausbeute.

Beispiele 3 bis 14

Reduktion weiterer Verbindungen

Auf die in Beispiel 1 oder Beispiel 2 angegebene Weise wurden weitere Verbindungen reduziert. Einzelheiten zu diesen Beispielen sind der Tabelle zu entnehmen, in welcher außerdem die Beispiele 1 und 2 veranschaulicht wurden.

Tabelle - Reduktion verschiedener Verbindungen (Edukte)

| Beispiel | Edukt | Produkt | Ausbeute (isoliert) % |
|---|---|---|---|
| 1[1] | 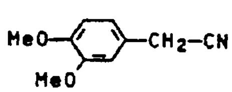 3,4-Dimethoxybenzylcyanid | 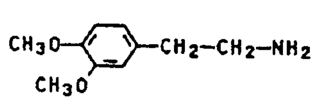 2-(3,4-Dimethoxyphenyl)-ethylamin | 90 |
| 2[2] | 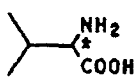 L-Valin | 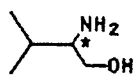 L-Valinol | 91 |
| 3[1] | Me—CN Acetonitril | Me—$CH_2$—$NH_2$ Ethylamin | 70 |
| 4[1] | Ph—CN Benzonitril | Ph—$CH_2$—$NH_2$ Benzylamin | 90 |

* = chirales Zentrum

[1] = Methode nach Beispiel 1

[2] = Methode nach Beispiel 2

Tabelle (Fortsetzung)

| Beispiel | Edukt | Produkt | Ausbeute (isoliert) % |
|---|---|---|---|
| 5[1)] | 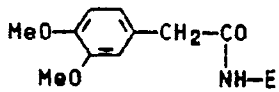<br>3,4-Dimethoxyphenyl-essigsäure-N-ethyl-amid | 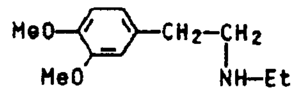<br>2-(3,4-Dimethoxyphenyl)-N-ethyl-ethylamin | 90 |
| 6[1)] | $H-CO-NMe_2$<br>Dimethylformamid | $Me_3N$<br>Trimethylamin | 72 |
| 7[2)] | 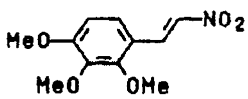<br>2-(2,3,4-Trimethoxy-phenyl)-nitroethen | 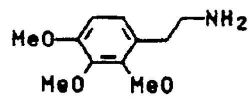<br>3-(2,3,4-Trimethoxy-phenyl)-n-propylamin) | 75 |
| 8[2)] | $Ph-CH_2-O-CO-NH-(CH_2)_6-OH$<br>N-(6-Hydroxy-n-hexyl)-benzylurethan | $H_2N-(CH_2)_6-OH$<br>Amino-n-hexan-6-ol | nicht ermittelt |
| 9[1)] | Me—SO—Me<br>Dimethylsulfoxid | Me—S—Me<br>Dimethylsulfid | rd. 60 |
| 10[2)] | 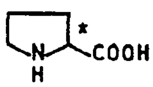<br>Prolin | 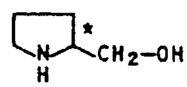<br>2-Methylolpyrrolidin | rd. 80 |
| 11[2)] | 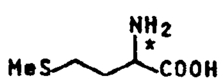<br>Methionin | 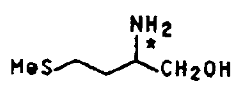<br>4-Mercapto-2-aminobutanol | 94 |
| 12[2)] | 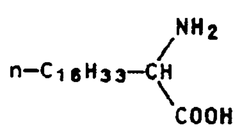<br>2-Aminostearinsäure | 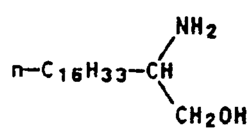<br>2-Aminooctadecanol | 85 |
| 13[2)] | Ph—COOH<br>Benzoesäure | $Ph-CH_2-OH$<br>Benzylalkohol | 92 |

Tabelle (Fortsetzung)

| Beispiel | Edukt | Produkt | Ausbeute (isoliert) % |
|---|---|---|---|
| 14[2)] | 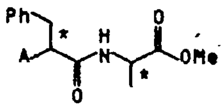 1-Fluorenyl-5-benzyl-8-methyl-4,7-diaza-2,10-dioxa-3,6,9-trioxo-undecan | 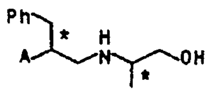 1-Fluorenyl-5-benzyl-8-methyl-4,7-diaza-2-oxa-3-oxo-nona-9-ol | 95 |

A = 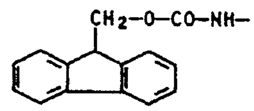

* = chirales Zentrum

Beispiel 15

Reduktion von Benzylcyanid

Eine Mischung aus 11 g (0,29 mol) Natriumboranat und 100 ml Tetrahydrofuran wurde bei 66°C im Laufe von 15 min mit 16,8 g (0,13 mol) Dichlordimethylsilan versetzt und weitere 60 min unter Rückflußkühlung erhitzt, wonach eine Lösung aus 7 g (0,06 mol) Benzylcyanid und 10 ml Tetrahydrofuran zugetropft wurde. Das Reaktionsgemisch wurde nach 3 h zum Sieden erhitzt und anschließend vorsichtig der Alkoholyse mit Methanol und dann der Hydrolyse unterworfen. Die übliche Aufarbeitung lieferte das 2-Phenylethylamin in 86%iger Ausbeute.

**Patentansprüche**

1. Verfahren zur Reduktion reduzierbarer Verbindungen mit Hilfe von Alkalimetallboranaten in Gegenwart eines komplexierenden Lösungsmittels und eines Reduktionshilfsmittels, dadurch gekennzeichnet, daß man als Reduktionshilfmittel wirksame Mengen eines Alkylhalogensilans der allgemeinen Formel I

$$R_n\text{-Si-}X_{4-n} \quad \text{I}$$

mitverwendet, in der R für gleiche oder verschiedene $C_1$-$C_4$-Alkylgruppen steht, X Chlor oder Brom bedeutet und n einen Wert von 1 bis 3 hat.

2. Verfahren nach Anspruch 1, dadurch gekennzeichnet, daß man als Alkylhalogensilan Trimethylchlorsilan verwendet.

3. Verfahren nach Anspruch 1, dadurch gekennzeichnet, daß man als Alkylhalogensilan Dimethyldichlorsilan verwendet.

**Claims**

1. A process for reducing reducible compounds using alkali metal borates in the presence of a complexing solvent and in the presence of a reduction aid, wherein, as reduction aid, effective amounts of an alkylhalosilane of the formula I

$$R_n\text{–}Si\text{–}X_{4-n} \quad I$$

where the radicals R are identical or different $C_1$-$C_4$-alkyl groups, X is chlorine or bromine and n has a value from 1 to 3 are used.

2. A process as claimed in claim 1, wherein the alkylhalosilane used is trimethylchlorosilane.

3. A process as claimed in claim 1, wherein the alkylhalosilane used is dimethyldichlorosilane.

## Revendications

1. Procédé de réduction de composés réductibles à l'aide de boranates de métaux alcalins et en présence d'un solvant de complexation et d'un agent réducteur, caractérisé en ce qu'à titre d'agent réducteur, on utilise conjointement des proportions actives d'un halogénosilane de la formule générale I :

$$R_n\text{–}Si\text{–}X_{4-n} \quad I$$

dans laquelle R représente des radicaux alkyle en $C_1$-$C_4$, X représente du chlore ou du brome et n possède une valeur qui fluctue de 1 à 3.

2. Procédé selon la revendication 1, caractérisé en ce que l'on utilise le triméthylchlorosilane à titre d'alkylhalogénosilane.

3. Procédé selon la revendication 1, caractérisé en ce que l'on utilise le diméthyldichlorosilane à titre d'alkyl-halogénosilane.